# EUROPEAN PATENT APPLICATION

(11) **EP 3 117 825 A1**
(43) Date of publication of application: **18.01.2017**
(21) Application number: 15002128.5
(22) Date of filing: 16.07.2015
(51) Int. Cl.: A61K 31/045, A61K 31/445, A61K 31/4745, A61K 31/519, A61K 36/062, A61K 36/29, A61K 36/60, A61P 3/04, A61P 3/06

(54) **ORAL FORMULATION COMPRISING BERBERINE AND MORUS ALBA EXTRACT**

(71) Applicant: Rottapharm S.p.A., 20900 Monza (IT)
(72) Inventor: The designation of the inventor has not yet been filed
(74) Representative: Endler, Gabriele

(57) **Abstract**

The present invention relates to a formulation for oral administration, in the form of tablets, capsules or of powder for extemporaneous use, which is able to exert a beneficial effect on the cardiovascular system and for treating excess of weight comprising Berberine and Morus alba extract.

## Description

The present invention relates to a formulation for oral administration, in the form of tablets, capsules or of powder for extemporaneous use, which is able to exert a beneficial effect on the cardiovascular system and for treating excess of weight, owing to a combination of activities such as eulipidaemic, euglycaemic, anti-stress and anti-fatigue, cholesterol-lowering and triglyceride-lowering, blood pressure normalizing, antioxidant, cardioprotective and protective of the vasal endothelium and others connected with these directly or indirectly e.g. by reduction of ROS (Reactive Oxygen Species) and increased hepatic transaminases, balancing increased risk of smokers or patients with high homocysteine or fibrinogen levels or of imbalanced diet.

In the industrialized countries, cardiovascular diseases are among the main causes of death for men and women [Schwarz et al. 1999, Lowe et al. 1998, Sempos et al. 1993] and in this connection, extensive epidemiological studies have identified the conditions that predispose to cardiovascular diseases, i.e. the so-called "risk factors" which can be classified as "unalterable" (genetic, sex, age) and "alterable". The alterable factors are mainly metabolic factors (for example hypercholesterolaemia, hypertriglyceridaemia, hyperfibrinogenaemia, hyperhomocysteinaemia, diabetes mellitus, visceral obesity), biological factors (for example arterial hypertension) and factors connected with lifestyle (smoking, incorrect eating habits, sedentary lifestyle, etc.).

Many cardiovascular diseases are of an arteriosclerotic nature, i.e. are due to narrowing of the lumen of the arteries owing to the deposition of cholesterol, calcium and fibrin in the vessel wall, or to occlusion of the arteries caused by a thrombus that formed from an atheromatous plaque. As was documented in the Framingham epidemiological study [Kannel et al. 1995], the incidence and the severity of arteriosclerosis are closely related to the cholesterol level in the blood and especially when low-density lipoproteins (LDL), or "bad" cholesterol, exceed values of 160 mg/dl [Ministry of Health 2003]. In fact it is now generally agreed that coronary pathology might be effectively prevented by maintaining plasma cholesterol at levels below 190 mg/dl. There are, however, relatively few adults who have a blood cholesterol level below the aforementioned limit. A study conducted by the Italian Cardiovascular Epidemiological Centre [2004] shows that in fact 6 out of 10 Italians have a cholesterol level above the aforesaid threshold.

It is therefore very important to keep blood cholesterol under control, as is also recommended by the "European guidelines on cardiovascular prevention" [2003]. In practice, if there is mild or moderate hypercholesterolaemia (blood cholesterol between 190 and 250 mg/dl), a change of lifestyle is required, adopting a healthy diet, low in saturated fats and rich in fruit and vegetables, with a calorie intake adjusted to the individual's needs. It is also necessary to eliminate unnecessary risk factors, especially smoking, and ensure adequate physical activity every day.

These are simple rules but they are not easy to follow in present living conditions. Dietary supplements that act as adjuvants in the control of plasma cholesterol can therefore provide useful support, in conjunction with a generally adequate diet.

Another important cardiovascular risk factor is represented by raised levels of triglycerides (above 150-200 mg/dl), especially if accompanied by a reduced HDL cholesterol level (< 40 mg/dl) or within the so-called "metabolic syndrome".

Another pathogenetic factor of arteriosclerosis is peroxidation of LDL. In fact LDLs are transported in the blood without particular pathological problems. However, if they are peroxidized by ROS (Reactive Oxygen Species); they pass through the endothelium of the arteries and are captured and stored by macrophages. The latter, packed with peroxidized LDL, are transformed to so-called "foam cells", which represent the initial nucleus of atheromas of the intima, from which arteriosclerotic plaques then form. Antioxidants that inhibit the peroxidation of LDL are therefore very useful for preventing arteriosclerosis. This objective can be achieved with an appropriate choice of foods and with the support of suitable dietary supplements rich in antioxidants.

Then there are other metabolic factors of cardiovascular risk, among which we should certainly mention homocysteine, which is an atherogenic and thrombogenic agent. Increase in plasma homocysteine (hyperhomocysteinaemia) represents an independent risk factor for diseases of coronary, cerebral or peripheral vascular origin, or originating from deep vein thrombosis [Longo 2001]. The relation between hyperhomocysteinaemia, i.e. blood homocysteine above 10 µmol/l, and increased cardiovascular risk was examined and demonstrated in a thorough meta-analysis carried out on 27 controlled prospective clinical studies [Boushey et al. 1995]. It is therefore necessary to avoid hyperhomocysteinaemia, for example with the administration of B-group vitamins and in particular folic acid.

As is known, according to the World Health Organisation (WHO) overweight and, especially, obesity represent one of the biggest public health problems in the world. In recent years there has been a global epidemic, which is taking over many parts of the world. If immediate action is not taken it will cause serious health disorders in the coming years.

Obesity and overweight are associated with premature death and are now universally recognised as risk factors for the main chronic diseases: cardiovascular disease, stroke, diabetes, certain forms of cancer (of the endometrium, colon and rectum, kidney, gallbladder and post-menopause breast cancer), gallbladder disorders, osteoarthritis.

Another important problem, of increasing importance not only in industrialised countries, is the high risk of diabetes among people who are overweight.

A further serious problem of increasing importance is the incidence of obesity among children and adolescents, who are exposed, from a very early age, to the risk of developing respiratory illnesses, joint and movement problems, but also psychological disorders and problems with digestion.

Moreover, children who are overweight tend to grow into overweight adults, and are thus more likely to develop risk factors for cardiovascular disease (hypertension, coronary disease, risk of heart attack) and metabolic disorders while still young.

In view of the problems mentioned above and the importance of maintaining a correct body weight, more and more medicines, supplements and other similar elements that permit people to lose weight are being developed.

At present, one of the most effective methods for helping people lose weight envisages the use of drugs known as "slimming tablets" made of caffeine, ephedrine or amphetamine which raise the rate of metabolism and thus increase the calories burned.

While having important advantages, these drugs also have some no less important contraindications.

The use of amphetamines can lead to numerous problems such as, for example, dehydration, gastrointestinal disorders, headache, hypertension, arrhythmias, angina pectoris, infarction, cardiac valve disease, pulmonary hypertension, increased risk of stroke.

Another problem, related to the use of said pills, consists of the fact that the body gradually builds up a sort of resistance to the slimming effects of the dug making it necessary to increase the dose and consequently the risks associated with the use of amphetamines. The use of amphetamines and similar substances leads to dependence and addiction which, when the treatment is stopped, cause mental depression and fatigue.

For these reasons, other drugs have been developed which exploit other active ingredients that can help people to lose weight.

One of the most important drugs is Sibutramine® which acts on the central nervous system exploiting a mechanism of action similar to that of some antidepressant drugs. In particular, it inhibits the synaptic reuptake of norepinephrine, serotonin and, to a lesser extent, dopamine and thus promotes a natural feeling of satiety after meals.

Another type of drug, such as Orlistat®, for example, acts upon the digestive tract.

In particular, Orlistat® reduces the absorption of dietary fat preventing the absorption and breakdown of triglycerides by inhibiting gastric lipases, namely the enzymes that break triglycerides down into simpler fragments more easily absorbed by the intestinal mucosa (fatty acids and monoglycerides).

Orlistat® also has some important drawbacks.

A first important drawback lies in the fact that the undigested triglycerides lead to the problems typically associated with steatorrhea and, in some cases, flatulence, incontinence, excess fat in the faeces and faecal urgency.

There are at present various dietary supplements that can provide beneficial action and give a significant reduction of cardiovascular risk and excess of weight, especially if combined with a controlled diet and with healthy physical activity.

A scientific work was published recently [Setnikar et al. 2005], subsequent to an Italian patent application [application TO2004A000682], which describes a composition for oral administration based on policosanol, red yeast (RYR) and an antioxidant selected from astaxanthin and folic acid, which clearly shows eulipidaemic efficacy.

The present invention is based on the development of a composition for oral administration in which an active principle that has effective and demonstrable triglyceride-lowering action and at the same time is useful for treating excess of weight and substantially has no contraindications and can thus be used by practically everyone. The choice fell on berberine, a natural substance of vegetable origin whose triglyceride-lowering and cholesterol-lowering activities were recently documented clinically [Kong et al. 2004] and second active component comprises an active ingredient defined by 1-deoxynojirimycin (DNJ), a natural extract obtained from blackberries, the silkworm or the white mulberry (*Morus alba* extract). The combination of these two active ingredients surprisingly shows a synergistic effect in weight reduction.

The invention thus relates to a composition for oral administration as defined in the claims that follow.

The properties of the various active components, the object of the present invention, together with those of other potential synergists such as folic acid, are described hereunder.

### White Mulberry Leaf Extract (Morus alba)

In traditional Oriental medicine this plant is used as a remedy for diabetes, it contains 2% of 1-deoxynojirimycin (DNJ) a piperidine present in the extract of the bark and in the leaves of White Mulberry *(Morus alba*).

### Chemistry

Chemically DNJ is (2*R*,3*R*,4*R*,5*S*)-2-(hydroxymethyl)piperidine-3,4,5-triol.

### Mechanism of action

The DNJ is known to be a potent inhibitor of intestinal sucrase and isomaltase because it slows down the conversion of carbohydrates to monosaccharides, thereby delaying absorption and reducing significantly the response of the rise in blood glucose after lunch.

Scientific studies show that DNJ inhibits the intestinal absorption of glucose and accelerates the hepatic metabolism of glucose, adjusting directly the expression of proteins involved in the transport systems of the glucose and enzymes of glycolysis gluconeogenesis.

It's also showed a beneficial change in the profile of lipoproteins administering extracts rich in mulberry DNJ for 12 weeks.

### Posology

The daily dose used in the invention is preferably 50 - 1000 mg (usually 300 mg), preferably taken after the evening meal.

### Berberine

Berberine is a natural vegetable preparation extracted from the bark of *Berberis aristata,* a spiny shrub originating from the Himalayas and Nepal, belonging to the Berberidaceae family.

### Chemistry

Chemically berberine is 5,6-Dihydro-9,10-dimethoxybenzo[g]-1,3-benzodioxolo[5,6-a]quinolizinium with the empirical formula C₂₀H₁₀NO₄⁺ and molecular weight 336.37. Berberine is obtained from the bark of *Berberis aristata* by aqueous-alcoholic extraction and then crystallization, processes which leave the chemical structure of the molecule unchanged, whereas microbial contamination is kept within the limits stipulated by the European Pharmacopoeia for pharmaceutical use.

### Eulipidaemic effect

Berberine is used traditionally for its antimicrobial action in infectious diarrhoea, in urinary tract infections and for local treatment of wounds and ulcers. In addition it has immunostimulant, antipyretic and antihaemorrhagic action [Leung et al. 1989] [Monograph 1989].

A controlled clinical study on 91 hypercholesterolaemic patients, 63 of whom were treated for 3 months b.i.d. with 500 mg of berberine, and 28 with placebo, was published just recently [Kong et al. 2004]. The patients treated with berberine showed a significant decrease of 29% in total cholesterol (2% with placebo), 25% in low-density lipoproteins (LDL) (0% with placebo) and 35% in triglycerides (5% with placebo). HDL, in contrast, remained unchanged.

Another controlled study was conducted on 20 patients who had hypercholesterolaemia and hypertriglyceridaemia, administering 500 mg of berberine in a single dose once a day [Cicero et al. 2006]. Berberine reduced total cholesterol by 16%, LDL by 20% and triglycerides by 22%. All these changes were found to be statistically highly significant (p 0.0001). Moreover, HDL ("good" cholesterol) increased by 7% (p <0.05). The treatment was well tolerated and hepatic transaminases were improved.

These two clinical Studies demonstrate that berberine exerts an effective eulipidaemic spectrum on various blood lipids and suggest that it can be used in the prevention of cardiovascular risks connected with atherosclerotic manifestations.

The eulipidaemic action of berberine was further confirmed on hamsters fed a diet enriched with cholesterol and fats, which caused a large increase in blood levels of total cholesterol, LDL and triglycerides [Kong et al. 2004]. Treatment with 50 and with 100 mg/kg/d of berberine for 10 days significantly reduced, by 25% and 38% respectively, the increase in total cholesterol caused by the hyperlipidic diet and, by 26% and 41% respectively, the increase in LDL.

### Mechanism of action

Investigation of the molecular mechanism of action showed that berberine increases, in the liver, mRNA and the LDL receptor (LDLR) protein, involving an extracellular signal-regulated kinase (ERK). Furthermore, berberine stabilizes the LDLR and prolongs its half- life. With these mechanisms, berberine causes a notable increase in LDLRs located on the membrane of the hepatocytes, promoting endocytosis of the LDLs and hence their metabolic elimination. The mechanism by which berberine reduces the blood LDL is therefore different and independent of that of the statins, which act by inhibiting the endogenous synthesis of cholesterol from mevalonate by antagonizing 3-hydroxy-3-methylglutaryl-coenzyme A (HMG-CoA) reductase.

One interesting result of this study is the ability of berberine to lower the triglyceride level and therefore exert an overall eulipidaemic action.

Another interesting result is the decrease in hepatic transaminases in the patients treated with berberine 500 mg b.i.d.

In animals fed with the hyperlipidic diet, moreover, berberine is able to reduce the hepatic steatosis caused by the hyperlipidic diet.

### Posology

The daily dose used in the invention is preferably 125 - 500 mg (usually 250 mg), preferably taken after the evening meal.

### Policosanol

### Chemistry

"Policosanol" is the generic name given to a mixture of saturated long-chain (C₂₂-C₃₆) primary aliphatic alcohols, in the form of a solid, of waxy consistency, sparingly soluble in H₂O. The alcohols of which it is composed occur in the natural state in beeswax (from *Apis mellifera*), in the waxy matrix of sugar cane *(Saccharum officinarum*), in rice bran (*Oryza sativa*) and in various other plants.

The policosanol used in the present invention, in contrast to that normally obtained with the conventional wet processes which do not guarantee its purity and the total removal of the organic solvents employed in the extraction process, is preferably extracted by means of carbon dioxide in the supercritical state at extremely low temperature in the liquid phase. This process guarantees its purity, conservation of the original chemical structure, total absence of organic solvents and preservation of the original proportions of the alcohols naturally present in the starting product.

### Eulipidaemic effect

Numerous clinical and experimental studies have documented the eulipidaemic action of policosanol, which is especially evident in subjects with hypercholesterolaemia. The effect is most noticeable on cholesterol associated with low-density lipoproteins (LDL-C) with inhibition of the synthesis of the cholesterol itself and increase in its elimination [Menendez et al. 1994, Menendez et al. 1996, Menendez et al. 2001].

Investigations into the mechanism of action of policosanol provide evidence of inhibition of the biosynthesis of cholesterol in a stage that is positioned between utilization of the acetate and production of the mevalonate, but excluding a direct effect on 3-hydroxy-3-methylglutaryl coenzyme A reductase (HMG-CoA-r) [Menendez et al. 1996, Menendez et al. 2001] on which other well-known cholesterol-lowering agents act, for example the statins.

Policosanol, moreover, increases significantly, and in a dose-dependent manner, the binding and hepatic uptake of the LDLs, with a consequent positive acceleration of their catabolism [Menendez et al. 1994]. For policosanol, an action on the intestinal mucosa has also finally been proposed, relating to the absorption and intracellular metabolism of food lipids [Goumi-Berthold et al. 2002].

### Inhibition of LDL peroxidation

Various studies in vitro and in vivo have demonstrated that policosanol exerts an antioxidant action with consequent inhibition of the peroxidation of LDLs and of VLDLs (very low density lipoproteins) [Menendez et al. 1999]. Blocking of the peroxidation of LDLs, also confirmed by human clinical trials [Menendez et al. 2000], leads to an important antiatherogenic effect, because the LDLs only exert their atherogenic action in the oxidized state. This is in fact a necessary structural condition so that the LDLs can filter through the endothelium of the arteries to be captured by the receptor for the scavengers of the macrophages. The macrophages filled with peroxidized LDLs are transformed into "foam cells", the first step in the formation of atheromatous plaques.

### Endothelial protection

Policosanol protects the vasal endothelium from damage caused by mechanical and chemical agents [Benitez et al. 1997]. This epithelial damage leads to loss of membrane elasticity and an increase in permeability, which among other things promotes passage of the LDLs from the blood to the intima of the artery, with resultant formation of atheromatous plaques.

### Pro-energetic action

Clinical research conducted since the 1950s has demonstrated that the use of policosanol brings beneficial effects on resistance to stress and to fatigue. This action involves the bioavailability of muscle glycogen, nerve reaction times, resistance to stress from hypoxia, the oxygen debt and, not least, the blood cholesterol level [Cureton 1972].

Policosanol is therefore used by athletes and sports people in general for its pro-energetic effect, which would be reflected in its role of "intracellular vehicle" for the essential fatty acids and for the other energy sources of the cell.

### Posology

The daily dose used within the scope of the invention is preferably 2,5 - 20 mg (usually 5 - 10), preferably taken with the evening meal to obtain optimum absorption of the fatty alcohols. To be effective, the duration of treatment should be extended to at least six weeks.

### Red yeast (RYR)

### Chemistry

RYR, or fermented red rice, is the product of fermentation of rice by a fungus, *Monascus purpureus,* which produces various substances, including a red pigment (whence the name "red yeast"), bacteriostatic agents and substances that modulate the level of lipids in the blood. The latter are in fact the most interesting components for their beneficial effect on health. They are identified with the name "monacolines". The monacoline that is most effective for eulipidaemic purposes is monacoline K [Endo 1988] the content of which in RYR depends on the strain of *Monascus purpureus* used and the fermentation conditions.

The RYR used in the formulation that is the object of the present invention is preferably obtained in standardized fermentation conditions, with a particular strain of *Monascus purpureus,* selected for an optimum yield of monacoline K and a defined and titrated content of said active principle.

### Eulipidaemic effect

The blood cholesterol level is the sum of that ingested with food and of that arising from endogenous biosynthesis in the liver. This synthesis starts out from acetyl coenzyme A, from which 3-hydroxy-3-methylglutaryl coenzyme A (HMG-CoA) forms, and then, with the intervention of HMG-CoA reductase (HMG-CoA-r), mevalonate. Mevalonate is the key intermediate for the next stages of the cascade in the biosynthesis of cholesterol and represents the critical, limiting step for the endogenous production and consequent blood levels of cholesterol itself and of the LDLs.

Monacolin K competes structurally at the level of HMG-CoA-r with HMG-CoA, the mevalonate precursor, and has an activity for the enzyme 2000 times greater than that of the natural substrate, therefore giving rise to a mechanism of "competitive enzymatic inhibition" which impedes the biosynthesis of cholesterol [Man et al. 2002].

### Posology

The daily dose of RYR used within the scope of the invention is preferably the equivalent of an amount of monacolin between 1 and 6 mg, preferably 1,5 mg and preferably does not exceed the equivalent of 3 mg of monacolin in the compositions for use as nutritional supplement according to the invention.

### Folic acid (FA)

Chemistry FA comprises a combination of three molecules: glutamic acid, p-aminobenzoic acid and a derivative of pteridine.

Our body is not able to synthesize FA and we are therefore dependent on food sources that are rich in it, such as leafy vegetables, liver, eggs and legumes. The daily requirement for FA, recommended for adults, is 0.2 mg and increases to 0.4 mg in pregnancy and to 0.3 mg during lactation.

### Hyperhomocysteinaemia correcting effect

FA performs an essential role in many biological functions, such as synthesis of the nucleic acids DNA and RNA, the production and development of the red blood cells, proper development of the embryo in pregnancy, improvement of trophism and of the health of the skin, the stimulation of milk secretion, regulation of iron metabolism etc.

Moreover, FA corrects hyperhomocysteinaemia, a cardiovascular risk factor only a little less important than that of hypercholesterolaemia for coronary, cerebral and peripheral vascular diseases and for deep vein thrombosis [Longo 2001]. The action of FA is connected with its property as a donor of methyl groups which convert homocysteine to L-methionine and S-adenosylmethionine, removing excess homocysteine in the blood.

### Posology

The dosage forms of the invention are preferably arranged for the administration of folic acid at daily doses from 0.1 to 0.6 mg, and more preferably from 0.1 to 0.2 mg.

### Examples

The formulations reported below illustrate possible practical applications of this invention and as such must not in any way be regarded as limiting the invention.

In particular the composition according to the invention may contain effective quantities from 150 mg to 2000 mg of berberine, from 10 to 500 mg of *Morus alba,* from 1 mg to 10 mg of Red Yeast Rice in daily doses for oral administrations.

As far as excipients are concerned, other excipients may be used as an alternative to those explicitly mentioned in this invention, dependent on formulation and technological requirements, and in any event may be selected from a very wide range of products available on the market and well known to those skilled in the pharmaceutical art and therefore without any inventive originality.

### Example 1: Tablets for oral use

| | |
|---|---|
| Microcrystalline cellulose | 175 |
| Magnesium Stearate | 6 |
| Silicon Dioxide | 8 |
| Berberine Chloride | 320 |
| Red Yeast Rice | 30 |
| *Morus alba* | 300 |
| Policosanol | 5.5 |
| Carboxymethylcellulose | 25 |
| Crosslinked PVP | 30 |
| Total: | 900 |

### Example 2: Heat-sealed sachets containing powder for extemporaneous

| | |
|---|---|
| Maltodextrine | 600 |
| Silicon Dioxide | 10 |
| Berberine Chloride | 320 |
| Red Yeast Rice | 30 |
| *Morus alba* | 300 |
| Policosanol | 5.5 |
| Sorbitol | 600 |
| Total: | 1865.5 |

## Claims

1. Composition for oral administration for treating excess of weight, comprising as active principles a policosanol or a mixture of policosanols, red yeast, folic acid, berberine or an extract containing berberine, **characterized in that** it further comprises an active ingredient defined by 1-deoxynojirimycin *(Morus alba* extract) suitable to control glycaemic metabolism.

2. Composition as claimed in claim 1, wherein said 1-deoxynojirimycin is naturally extracted.

3. Composition as claimed in claim 1, wherein said 1-deoxynojirimycin is extracted from blackberries.

4. Composition as claimed in claim 1, wherein said 1-deoxynojirimycin is extracted from the silkworm.

5. Composition according to any one of Claims 1 to 4, in the form of a unit dose, ready for the administration of berberine at a daily dose from 125 to 1000 mg.

6. Composition according to any one of Claims 1 to 5, in the form of a unit dose, ready for the administration of policosanols at a daily dose from 2,5 to 50 mg, preferably from 5 to 10 mg.

7. Composition according to any one of Claims 1 to 6, in the form of a unit dose, ready for the administration of red yeast at a daily dose corresponding to 1 to 6 mg of monacoline, preferably 1,5 mg, preferably not more than 3 mg of monacoline.

8. Composition according to any one of Claims 1 to 8, in the form of a unit dose, ready for the administration of folic acid at a daily dose from 0.1 to 0.6 mg, and preferably from 0.1 to 0.2 mg.

9. Composition according to any one of Claims 1 to 8, in the form of a unit dose, ready for the administration of 1-deoxynojirimycin at a daily dose from 50 to 1000 mg, preferably from 200 to 400 mg.

10. Composition according to any one of the preceding claims, in the form of tablets, capsules or powder for extemporaneous suspension.
